# EUROPÄISCHE PATENTANMELDUNG

(11) **EP 4 328 588 A1**
(43) Veröffentlichungstag der Anmeldung: **28.02.2024**
(21) Anmeldenummer: 23192680.9
(22) Anmeldetag: 22.08.2023
(51) Int. Cl.: G01N 33/543, G01N 1/14, G01N 33/02, G01N 33/487

(54) **VORRICHTUNG ZUR DETEKTION MINDESTENS EINES ANALYTEN IN EINER PROBE**

(30) Priorität: 22.08.2022 DE 102022121185
(71) Anmelder: Digid GmbH, 55129 Mainz (DE)
(72) Erfinder: Kloppstech, Konstantin, Dr., 55129 Mainz (DE); Könne, Nils, Dr., 55129 Mainz (DE); Von Gersdorff, Constantin, 55129 Mainz (DE)
(74) Vertreter: Nordmeyer, Philipp Werner

(57) **Zusammenfassung**

Die vorliegende Erfindung betrifft eine Vorrichtung (1) zur Detektion mindestens eines Analyten (90) in einer Probe (9), umfassend mindestens eine Kapillare (3), mindestens eine Messkammer (2) und mindestens ein Cantileverpaar (4), wobei die Kapillare (3) dazu eingerichtet ist, eine Probe (9) zu entnehmen und der Messkammer (2) zuzuführen, wobei die Messkammer (2) dazu eingerichtet ist, die Probe (9) zu sammeln und dem Cantileverpaar (4) zur Verfügung zu stellen, und wobei das Cantileverpaar (4) einen Referenzcantilever (42) und einen Testcantilever (40) umfasst, wobei der Testcantilever (40) zur selektiven Aufnahme des Analyten (90) aus der Probe (9) eingerichtet ist und wobei der Referenzcantilever (42) zur selektiven Nichtaufnahme des Analyten (90) aus der Probe (9) eingerichtet ist, wobei die selektive Aufnahme des Analyten (90) durch den Testcantilever (40) und die selektive Nichtaufnahme durch den Referenzcantilever (42) eine relative Verformung und/oder eine relative Veränderung der Oberflächenspannung des Testcantilevers (40) zum Referenzcantilever (42) verursacht, und wobei durch die relative Verformung und/oder die relative Veränderung der Oberflächenspannung des Testcantilevers (40) zum Referenzcantilever (42) der Analyt (90) detektiert wird.

## Beschreibung

### Technisches Gebiet

Die vorliegende Erfindung betrifft eine Vorrichtung zur Detektion mindestens eines Analyten in einer Probe.

### Stand der Technik

Die Verwendung von Federelementen beziehungsweise Cantilevern zur Detektion von Analyten in Proben ist bekannt. Hierbei wird die Wechselwirkung von Cantilevern mit einer Probenflüssigkeit und die Bindung des Analyten in der Probe an eine Beschichtung des Cantilevers ausgenutzt, um eine Verformung oder eine Veränderung der Oberflächenspannung des Cantilevers hervorzurufen. Aus der Verformung oder der Veränderung der Oberflächenspannung kann dann über einen Dehnungsmessstreifen auf das Verformen des Cantilevers durch den Analyten und somit mittelbar auf das Vorkommen des Analyten geschlossen werden.

Die Verformung von Cantilevern durch unterschiedliche Oberflächenspannungen ist beispielsweise in Rasmussen, P. A., Hansen, O., & Boisen, A. (2005). Cantilever surface stress sensors with single-crystalline silicon piezoresistors. Applied Physics Letters, 86(20), 203502. https://doi.orq/10.1063/1.1900299 beschrieben.

Die Detektion eines Analyten in einer Probe kann sich jedoch aufgrund mechanischer Störeinflüsse durch die Probenflüssigkeit und wegen weiterer chemischer Substanzen in der Probenflüssigkeit als schwierig erweisen, so dass es notwendig ist, aus dem eigentlichen Messignal die Anteile zu extrahieren, die lediglich auf die Wechselwirkung des Analyten mit dem Cantilever zurückzuführen sind.

Zudem wird, wenn der Analyt regelmäßig in einer Körperflüssigkeit detektiert werden soll, beispielsweise zur permanenten Untersuchung eines Patienten und zum Monitoring seiner Körperwerte, eine Vorrichtung benötig, die einerseits neue Probenflüssigkeit zuführen kann, um eine Zeitauflösung der Körperwerte zu ermöglichen und andererseits möglichst ohne operativen Eingriff an dem Patienten befestigt werden kann.

Aus der EP 297 226 4 und US 2021 321 942 sind tragbare Pflaster mit Mikronadeln bekannt, welche die Haut durchdringen und wobei verschiedene Analyten aus der Interstitialflüssigkeit mittels elektrochemischer Messungen bestimmt werden können.

Aus der EP 185 080 3 und der WO 111 433 24 sind implantierbare Sensoren bekannt, welche die Verformung funktionalisierter Cantilever bei der Wechselwirkung mit einem Analyten detektieren.

### Darstellung der Erfindung

Ausgehend von dem bekannten Stand der Technik ist es eine Aufgabe der vorliegenden Erfindung, eine verbesserte Vorrichtung zur Detektion eines Analyten in einer Probe bereitzustellen.

Die Aufgabe wird durch eine Vorrichtung zur Detektion eines Analyten in einer Probe mit den Merkmalen des Anspruchs 1 gelöst. Vorteilhafte Weiterbildungen ergeben sich aus den Unteransprüchen, der Beschreibung und den Figuren.

Entsprechend wird eine Vorrichtung zur Detektion mindestens eines Analyten in einer Probe vorgeschlagen, umfassend mindestens einer Kapillare, mindestens eine Messkammer und mindestens ein Cantileverpaar, wobei die Kapillare dazu eingerichtet ist, eine Probe zu entnehmen und der Messkammer zuzuführen, wobei die Messkammer dazu eingerichtet ist, die Probe zu sammeln und dem Cantileverpaar zur Verfügung zu stellen und wobei das Cantileverpaar einen Referenzcantilever und Testcantilever umfasst, wobei der Testcantilever zur selektiven Aufnahme des Analyten aus der Probe eingerichtet ist und wobei der Referenzcantilever zur selektiven Nichtaufnahme des Analyten aus der Probe eingerichtet ist, wobei die selektive Aufnahme des Analyten durch den Testcantilever und die selektive Nichtaufnahme durch den Referenzcantilever eine relative Verformung des Testcantilevers zum Referenzcantilever verursacht und wobei durch die relative Verformung des Testcantilevers und des Referenzcantilevers der Analyt detektiert wird.

Eine Probe bezeichnet hierbei eine beschränkte Menge eines Stoffes, die einer größeren Menge des Stoffes, etwa aus einem Reservoir, entnommen wurde, wobei die Zusammensetzung der Probe repräsentativ für die Zusammensetzung des Stoffs in dem Reservoir ist und dementsprechend aus dem Stoffvorkommen und Stoffzusammensetzungen der Probe auf das entsprechende Vorkommen im Reservoir geschlossen werden kann.

Beispielsweise kann eine Probe auch eine Körperflüssigkeit sein, insbesondere eine Zellflüssigkeit oder eine Interstitialflüssigkeit oder eine Blutprobe oder Schweiß sein. Die Probe kann auch eine Lymphflüssigkeit beziehungsweise Lymphe sein oder enthalten. Eine Probe kann aber auch eine nichtbiologische Probe, beispielsweise eine Probe eines chemischen Stoffs, sein. Die Probe enthält die chemische Information und/oder biochemische Information über den Analyten.

Ein Analyt ist hierbei der Stoff, dessen Vorliegen in der Probe qualitativ und/oder quantitativ nachgewiesen werden soll beziehungsweise mit dem Cantileverpaar detektiert werden soll. Der Analyt kann insbesondere unmittelbar in der Probe vorhanden sein, oder in der Probe gelöst sein oder der Probe oder einem Teil der Probe, insbesondere einem Probenpartikel, anhaften. Der Analyt kann mit der Probe auch eine chemische, biologische und/oder physikalische Wechselwirkung eingehen, sodass der Analyt lediglich indirekt über eine entsprechende Wechselwirkung mit dem Cantileverpaar, insbesondere mit dem Testcantilever, detektierbar ist. Beispielsweise kann ein Virus chemisch und/oder biologisch und/oder physikalisch so aufgeschlossen werden, dass eine Wechselwirkung eines dann resultierenden Analyten mit dem Cantileverpaar, insbesondere dem Testcantilever, möglich ist.

Die chemische Information kann beispielsweise die Art des Analyten, die Konzentration des Analyten, das Vorkommen des Analyten, das Gewicht des Analyten, die Reaktivität des Analyten, die Dichte des Analyten usw. umfassen. Die biochemische Information umfasst dieselben Eigenschaften wie die chemische Information, jedoch können diese Stoffe beispielsweise durch biologische Prozesse entstehen. Insbesondere spricht man von biochemischer Information, wenn der Analyt einen besonderen Einfluss auf den biologischen Kreislauf, beispielsweise den Stoffwechsel oder auf das Immunsystem hat.

Mit anderen Worten kann der Analyt ein Biomarker sein und die Probenflüssigkeit eine Körperflüssigkeit sein.

Die mindestens eine Kapillare kann dazu eingerichtet sein, die Probe aus dem Probenkörper zu entnehmen.

Beispielsweise kann die Kapillare zylindrisch oder spitzzulaufend sein und hohl ausgeformt sein oder die Form eines Strohhalms aufweisen. Insbesondere kann die Kapillare als Nadel oder Mikronadel ausgeformt sein. Bevorzugt kann die Kapillare stabil ausgeführt werden, um ein Brechen im oder am Probenkörper zu vermeiden.

Beispielsweise kann die Kapillare die Probenflüssigkeit mit einer konstanten Flussrate dem Probenkörper entnehmen. Die Flussrate kann beispielsweise über den Durchmesser der Kapillare besonders einfach eingestellt werden.

Zudem kann die Kapillare beschichtet sein, um die Adhäsion der Probenflüssigkeit oder in ihr enthaltener chemischer Spezies zu verringern oder um die Reibung mit dem Probenkörper zu verringern. Die Beschichtung kann zudem die Oberflächenenergie der Kapillare einstellen, um beispielsweise eine hydrophobe oder hydrophile Beschichtung zu erzeugen, oder eine Mischung daraus. Bevorzugt kann somit auch eine Proteinablagerung in oder auf der Kapillare vermieden werden oder verlangsamt werden.

Die Kapillare kann mit ihrem einen offenen Ende in einen Probenkörper oder ein Probenreservoir eindringen und von dort die Probenflüssigkeit zu ihrem zweiten Ende transportieren. Die Probenflüssigkeit kann anschließend in der Messkammer gesammelt werden.

Zu diesem Zweck kann die Kapillare einen Durchmesser von weniger als 200µm aufweisen, bevorzugt von weniger als 100µm aufweisen, und eine Länge zwischen 500µm und 5000µm aufweisen.

Beim erstmaligen Einsetzen der Vorrichtung muss die Messkammer mit Probenflüssigkeit gefüllt werden. Eine Möglichkeit hierfür ist, die Messkammer durch (mehrmaliges) Andrücken der Vorrichtung auf dem Probenkörper zu befüllen. Dazu ist die Vorrichtung bevorzugt entsprechend mechanisch so ausgebildet, dass ein gleichmäßiges Andrücken an den Probenkörper erreicht werden kann.

Durch den kleinen Durchmesser der Kapillare ist es möglich, dass die Kapillare besonders einfach in den Probenkörper eintreten kann. Die Länge der Kapillare ermöglicht es gleichzeitig, dass die Kapillare tief genug in den Probenkörper eintritt, so dass die Probenflüssigkeit in ausreichender Menge abtransportiert werden kann. Insbesondere kann, je nach Probenkörper, durch die Länge der Kapillare gesteuert werden, welche Probenflüssigkeit entnommen werden soll. Beispielsweise können kürzere Kapillaren eine Interstitialflüssigkeit eines menschlichen Probenkörpers entnehmen, während längere Kapillaren Blut aus dem Probenkörper aufnehmen.

Die Kapillare kann die Probe der Messkammer insbesondere über Diffusionskräfte und/oder osmotische Kräfte und/oder Kapillarkräfte zuführen.

Die Kapillarkraft basiert hierbei auf der Oberflächenspannung zwischen Probenflüssigkeit und Material der Kapillare. Dadurch ist es möglich, dass die Probenflüssigkeit ohne Aufwenden einer zusätzlichen externen Kraft durch die Kapillare entgegen der Schwerkraft gezogen wird.

Die Kapillarkraft kann durch eine spezifische Beschichtung der Kapillare eingestellt werden und auf diese Weise kann der Fluss durch die Kapillare eingestellt werden. Die Diffusionskraft erzeugt hierbei einen gerichteten Teilchen- beziehungsweise Flüssigkeitsstrom, der dazu bestrebt ist, das Konzentrationsgefälle eines Stoffes zwischen zwei verbundenen Reservoiren auszugleichen.

Beispielsweise kann über die Diffusionskraft ein konstanter Teilchenstrom erzeugt werden, wenn die Konzentration des Analyten auf einer Seite der Kapillare, beispielsweise in einem Probendepot z.B. auf Grund dessen Volumen (siehe unten) stets gering oder auf Null gehalten wird. Dadurch ist die Diffusionskraft immer bestrebt das Konzentrationsgefälle des Analyten auszugleichen, wobei die Konzentration in der Messkammer immer der Konzentration im Probenkörper entspricht.

Indem die Kapillare den Probenkörper mit der Messkammer verbindet, baut sich durch den Konzentrationsgradienten des Analyten ein Diffusionspotential auf, welches einen Teilchenfluss bewirkt. Zusätzlich wird die Probenflüssigkeit durch die Diffusionskraft durch die Kapillare geleitet, die beispielsweise von einem nanoporösen Probendepot (siehe unten) als kapillare Pumpe unterstützt werden kann. Die Diffusionskraft ist damit dazu geeignet geringe Mengen an Probenflüssigkeit in die Messkammer zu leiten, ohne einen Pumpvorgang aktiv durchzuführen. Die Kapillare kann einen konstanten Innendurchmesser aufweisen oder ein konisches Innenprofil aufweisen und insbesondere sich in Richtung des Messkammervolumens verjüngen. Bevorzugt kann die Kapillare durch eine gezielte Anpassung des Durchmessers einen Rückfluss der Flüssigkeit aus dem Messkammervolumen in den Probenkörper verhindern.

Die Probenflüssigkeit kann zusätzlich oder alternativ zu den oben genannten Mechanismen auch durch Konvektion, Drift oder durch eine extern eingebrachte Kraft wie beispielsweise ein elektrisches Potential oder ein Magnetfeld durch die Kapillare bewegt werden.

Die Probenflüssigkeit wird durch die Kapillare zur Messkammer transportiert. Die Messkammer kann hierbei ein Messvolumen umschließen, wobei das Messvolumen durch eine Probenzuleitungsfläche mit der mindestens einen Kapillare, einer daran anschließenden mindestens einen Messkammerwand und einem Messkammerdeckel begrenzt ist.

Die Messkammerwand kann hierbei einen Winkel zur Probenzuleitungsfläche einschließen, so dass die Messkammer ein gewisses Volumen erhält. Der Messkammerdeckel kann besagtes Messkammervolumen begrenzen und die Probenflüssigkeit im Messkammervolumen halten. Es ist aber auch möglich, dass der Messkammerdeckel semipermeabel ist, so dass die Probenflüssigkeit das Messvolumen nach einer gewissen Zeit wieder verlassen kann. Bevorzugt kann die Messkammerwand auch nanoporös sein und die Probenflüssigkeit nach außen ableiten.

Es kann auch mehr als eine Messkammer vorgesehen sein, wobei in jeder Messkammer mindestens ein Cantileverpaar oder einzelner Cantilever angeordnet ist. Durch das Vorsehen von mehr als einer Messkammer kann die über die Kapillare in die Messkammer geförderte Probenflüssigkeit unterschiedlich behandelt werden, beispielsweise chemisch und/oder biologisch und/oder physikalisch behandelt werden, um dann den oder die entsprechenden Analyten aufzuschließen, die dann mit dem Cantileverpaar, insbesondere dem Testcantilever, in Wechselwirkung treten. Durch das Bereitstellen von mehr als einer Messkammer kann entsprechend erreicht werden, dass unterschiedliche Behandlungen der Probenflüssigkeit durchgeführt werden können und auf diese Weise eine Mehrzahl an Analyten charakterisiert werden kann.

Zur Umwandlung der chemischen und/oder biochemischen Information des Analyten in ein elektrisches Signal umfasst die Vorrichtung einen Referenzcantilever und einen Testcantilever, die ein Cantileverpaar formen. Ein Cantilever ist hierbei ein Federelement, welches eine Basis und einen verformbaren Teil aufweist.

Die Basis ist hierbei ein unbeweglicher Teil des Cantilevers, der insbesondere ortsfest mit einem Substrat verbunden ist und/oder unterstützt wird und/oder aus dem Substrat herausgearbeitet ist. Die Basis der Cantilever ist als starre Basis ausgebildet, so dass lediglich der verformbare Teil des Cantilevers verformbar ausgebildet ist.

Der verformbare Teil des Cantilevers erstreckt sich in Längsrichtung über das Substrat, auf dem die Basis angeordnet ist, hinaus. Mit anderen Worten ist der verformbare Teil des Cantilevers einseitig an der Basis aufgehängt und nicht von dem Substrat unterstützt. Indem der verformbare Teil über das Substrat hinausragt, lässt sich der verformbare Teil des Cantilevers verbiegen, auslenken und dehnen. Die räumliche Grenze, ab der der Cantilever biegbar ist beziehungsweise der Cantilever von der Basis in den verformbaren Teil übergeht, wird Biegekante genannt. Die Biegekante ist üblicherweise eine Kante des Substrats, wenn der Cantilever über das Substrat hinausragt.

Wird der Cantilever verformt, so ergeben sich Materialspannungen und Kräfte in oder auf dem Material des Cantilevers, welche gemessen werden können. Sofern eine solche Materialspannung und/oder Kraft gemessen werden kann, lässt sich darüber auf eine Verformung des Cantilevers schließen. Eine Verformung kann eine anhebende oder absenkende Verformung sein. Der Cantilever kann sich aber auch selbst verformen, beispielsweise aufwölben, wellen oder verzerren.

Die Verformung der Cantilever kann durch eine geeignete Beschichtung stoffspezifisch herbeigeführt werden. Aus diesem Grund weist der Referenzcantilever eine Referenzschicht zur selektiven Nichtaufnahme des Analyten auf, während der Testcantilever eine Rezeptorschicht zur Aufnahme des Analyten aufweist.

Eine Rezeptorschicht ist hierbei ein Stoff, der mit dem Analyten in Wechselwirkung treten kann. Dies bedeutet wiederum, dass die Rezeptorschicht spezifisch für jeden Analyten gewählt wird. Analog ist eine Referenzschicht ein Stoff, der mit dem Analyten nicht in Wechselwirkung treten kann. Auch die Referenzschicht wird daher spezifisch für den Analyten gewählt.

Wechselwirkung bedeutet in diesem Fall, dass der Analyt in chemischer und/oder biochemischer und/oder physikalischer Wechselwirkung mit der Rezeptorschicht steht. Insbesondere kann die Wechselwirkung in einer Bindung des Analysten an die Rezeptorschicht bestehen. Eine Wechselwirkung kann außerdem in der Absorption oder Adsorption des Analyten an die Rezeptorschicht bestehen.

Die Rezeptor- und Referenzschichten bevorzugt chemisch identisch bezüglich möglicher Störeinflüsse und unterscheiden sich bevorzugt nur durch die Wechselwirkung mit dem Analyten. Ein Stoff, der nicht der Analyt ist, wechselwirkt dementsprechend genauso stark oder genauso schwach mit der Rezeptorschicht wie mit der Referenzschicht.

Die selektive Aufnahme des Analyten am Testcantilever bewirkt, dass eine Kraft durch den Analyten auf den Testcantilever wirkt, so dass der Testcantilever sensitiv auf den Analyten reagiert. Dementsprechend tragen die anderen Stoffe der Probe, die nicht der Analyt sind, lediglich zu einem Grundrauschen in Form einer Grundverbiegung am Testcantilever bei. Eine für die jeweilige Ausbildung mögliche Maximalkraft wird bei einer vollständigen Belegung des Cantilevers erreicht.

Die selektive Nichtaufnahme des Analyten am Referenzcantilever bewirkt hingegen, dass keine Kraft durch den Analyten auf den Referenzcantilever wirkt, so dass nur die Stoffe, die nicht der Analyt sind, zu einem Grundrauschen in Form einer Grundverbiegung des Referenzcantilevers beitragen.

Die durch die Anlagerung des Analyten an dem Testcantilever wirkende Kraft kann bei dem verformbaren Teil des Testcantilevers eine Verformung bewirken, während der verformbare Teil des Referenzcantilever nicht verbogen wird. Grundlage für die Auslenkung des Cantilevers ist die Änderung der Oberflächenspannung durch die Wechselwirkung mit dem Analyten. Die Änderung der Oberflächenspannung führt zu einer Dehnung oder Stauchung der oberen (oder unteren) Oberfläche des Cantilevers. Die unterschiedliche Dehnung oder Stauchung an Ober- und Unterseite bewirkt in dem Material eine interne Kraft oder Materialspannung, die zur Verformung führt.

Referenzcantilever nach dem Stand der Technik weisen lediglich keine Rezeptorschicht auf, die sensitiv auf den Analyten reagiert. Dadurch können zwar Effekte wie Turbulenz in der Probe und die thermische Drift, sowie pH-Wert und Konzentrationsvariationen der lonenkonzentration in der Probenflüssigkeit des Sensorsystems bestimmt werden. Jedoch kann bei einem solchen Referenzcantilever der Analyt beispielsweise durch eine unspezifische Bindung an die Referenzschicht des Referenzcantilevers binden. Dadurch trägt aber der Analyt selbst zum Grundrauschen bei. Daher sind bei einem Sensor nach dem Stand der Technik Referenzmessungen in einer Referenzprobe, also einer Probe ohne Analyten, notwendig. Nur dadurch lässt sich der Effekt der unspezifischen Bindung der Stoffe, die nicht der Analyt sind, feststellen.

Bei der erfindungsgemäßen Vorrichtung, wird durch die selektive Nichtaufnahme des Analyten durch den Referenzcantilever das Messverfahren drastisch vereinfacht, da der Referenzcantilever nicht sensitiv für den Analyten ist, und daher der Analyten auch nicht zum Grundrauschen beiträgt. Nur die Stoffe, die nicht der Analyt sind, tragen hier zum Grundrauschen des Referenzcantilevers bei. Gewissermaßen kann durch die selektive Nichtaufnahme des Analyten am Referenzcantilever bewirkt werden, dass der Referenzcantilever denselben Turbulenzen, derselben thermischen Drift und demselben Einfluss aller Stoffe, die nicht der Analyt sind, ausgesetzt ist, wie in einer Referenzflüssigkeit. Jedoch mit dem Unterschied, dass das Referenzsignal direkt in der Probenflüssigkeit bestimmt wird.

Insbesondere bewirkt ein Referenzcantilever mit Referenzschicht und ein Testcantilever mit Rezeptorschicht eine deutlich spezifischere Analyse des Analyten als lediglich ein Referenzcantilever ohne Rezeptorschicht, da sowohl die Referenzschicht als auch die Rezeptorschicht eine spezifische Wechselwirkung beziehungsweise Nicht-Wechselwirkung mit dem Analyten aufweisen.

Der Aufbau des Sensors mit Referenzcantilever und Testcantilever hat den Vorteil, dass in der Probe gleichzeitig zwei Messungen vorgenommen werden können, wobei die Messung des Referenzcantilevers die Messung des Testcantilevers kalibrieren kann. Dadurch lassen sich Umgebungseinflüsse, etwa chemische, thermische, mechanische, elektrische und fluidische Störeinflüsse, auf die jeweilige Messung reduzieren, so dass ein Vorkommen des Analyten aus dem Vergleich der Messung am Testcantilever und am Referenzcantilever geschlossen werden kann.

Diese Kräfte oder Materialspannungen, beispielsweise Dehnungen oder Stauchungen, die auf die Cantilever wirken, können schließlich von Transducern detektiert werden, wobei durch unterschiedlich starke Dehnungen oder Stauchungen unterschiedlich starke Spannungen von den Transducer detektiert werden.

Die Transducer haben demnach den Zweck, die Verformung der Cantilever zu bestimmen oder zu messen.

Bevorzugt sind die Transducer auf den verformbaren Teilen und/oder den Basen der Cantilever angeordnet. Beispielsweise kann eine Verformung des Cantilevers dazu führen, dass der Widerstand eines Transducers ansteigt oder abfällt, während hingegen keine Verformung des Cantilevers auch keine Veränderung des Widerstands des Transducers hervorruft. Dies kann beispielsweise über eine Ausbildung der Transducer nach dem Prinzip eines Dehnungsmessstreifens erfolgen, wodurch sich eine Verformung des jeweiligen Cantilevers in einer Längenänderung des darauf aufgebrachten Dehnungsmessstreifens des Transducers äußert und damit eine Verformung des Cantilevers direkt durch eine Veränderung des Widerstands des Dehnungsmessstreifens detektiert werden kann.

Somit wird die chemische und/oder biochemische Information des Analyten über einer Verformung des Cantilevers, eine anschließende Registrierung über einen Transducer, und schließlich über eine Änderung einer elektrischen Eigenschaft des Transducers detektierbar.

Indem beispielsweise erste und zweite Testtransducer auf den verformbaren Teilen des Testcantilevers angeordnet sind und erste und zweite Referenztransducer auf dem verformbaren Teil des Referenzcantilevers angeordnet sind, kann ein Maß der Dehnung der Testtransducers gefunden werden, welches der Stärke der Wechselwirkung des Analyten mit dem verformbaren Teil des Testcantilevers entspricht.

Indem beispielsweise erste Testtransducer auf den verformbaren Teilen des Testcantilevers angeordnet sind, zweite Testtransdurcer auf der Basis den Testcantilevers angeordnet sind, erste Referenztransducer auf dem verformbaren Teil des Referenzcantilevers angeordnet sind und zweite Referenztransdurcer auf der Basis des Referenzcantilevers angeordnet sind, kann ein Maß der Dehnung des ersten Testtransducers gefunden werden, welches der Stärke der Wechselwirkung des Analyten mit dem verformbaren Teil des Testcantilevers entspricht.

Beispielsweise kann der erste Referenztransducer des Referenzcantilevers durch Einfluss der Umgebungsbedingungen und Wechselwirkung mit der Probe einen ersten elektrischen Referenzzustand des ersten Referenztransducers hervorrufen, während die Wechselwirkung des Testcantilevers mit den Umgebungsbedingungen der Probe einen ersten elektrischen Testzustand des ersten Testtransducers hervorruft.

Beispielsweise kann der Referenzcantilever durch den Einfluss der Umgebungsbedingungen um einen ersten Betrag verbogen werden, sodass die Auslenkung in dem ersten Referenztransducer einen ersten Referenzzustand hervorruft und im zweiten Referenztransducer einen zweiten elektrischen Referenzzustand hervorruft, wohingegen der Testcantilever durch den Einfluss der Umgebungsbedingungen um einen zweiten Betrag verbogen wird und durch die zusätzliche Wechselwirkung mit dem Analyten in der Probe um einen dritten Betrag verbogen wird, was im ersten Testtransducer einen ersten elektrischen Testzustand hervorruft und im zweiten Testtransducer einen zweiten elektrischen Testzustand hervorruft.

Der Vergleich der elektrischen Zustände der ersten und zweiten Transducer gibt ein Maß für die Verformung der Cantilever an. Gleichzeitig ergibt ein Vergleich der jeweils ersten Transducer und/oder der jeweils zweiten Transducer ein Maß für die Unterschiedlichkeit der Verformung der Cantilever. Dadurch ist es möglich auf einen spezifischen Einfluss eines Analyten auf den Testcantilever zu schließen, bevorzugt den Einfluss zu quantifizieren und somit auf einen konzentrationsabhängigen Einfluss zu schließen.

Die Detektion des Analyten in der Probe kann kontinuierlich durchgeführt werden. Mit anderen Worten findet nicht nur eine einzige Detektion des Analyten statt, sondern die Detektion wird über einen Zeitraum hinweg durchgeführt. Bevorzugt wird die Detektion zeitaufgelöst durchgeführt, so dass Veränderungen im Vorliegen und/oder der Konzentration des Analyten über den Zeitverlauf hinweg detektiert werden können.

Dies ist insbesondere der Fall, wenn dem Messvolumen Probenflüssigkeit zugeführt wird und der Analyt an dem Testcantilever lediglich eine begrenzte Bindungszeit aufweist, die durch die Wahl der Testbeschichtung eingestellt werden kann. Nach der Bindungszeit löst sich der Analyt von der Testbeschichtung, so dass die zugehörige Bindungsstelle durch den Analyten der nachfolgenden Probenflüssigkeit aufgenommen werden kann.

Es ist aber auch möglich, dass die Testbeschichtung eine Kapazität von Bindungsstellen aufweist, die mit der Zeit immer weiter durch den Analyten der nachströmenden Probe besetzt werden. Demnach könnten sich die Cantilever immer weiter verbiegen, bis alle Bindungsstellen gebunden sind. Aus der zeitlichen Veränderung der Verbiegung oder der momentanen Verbiegung kann dann auf den Analyten geschlossen werden.

Dies hat den Vorteil, dass der Analyt, beziehungsweise dessen Vorkommen, zeitabhängig analysiert werden kann. Dies ist beispielsweise von Interesse, wenn bestimmte Vitalwerte im Probenkörper untersucht werden sollen, beispielsweise Lactat oder Glucose.

Beispielsweise kann durch eine kontinuierliche Messung der Analyt analysiert werden, ohne ihn chemisch zu verbrauchen, insbesondere chemisch umzuwandeln.

Bevorzugt kann die Cantilevergröße und somit die mögliche Wechselwirkungsfläche des Analyten mit dem Cantilever, auf die Flussgeschwindigkeit durch die Kapillare eingestellt werden. So kann beispielsweise der Effekt kleiner Schwankungen in der Fließgeschwindigkeit auf die Messung reduziert werden.

Das Cantileverpaar kann auf oder in der Messkammerwand angeordnet sein.

Durch ein Anordnen des Cantileverpaars auf der Messkammerwand kann erreicht werden, dass die Cantilever und die Messkammer konstruktiv getrennt werden, so dass beide Bauteile unabhängig voneinander gefertigt werden können. Insbesondere kann dadurch erreicht werden, dass die Cantilever zu der Probeneintrittsfläche der Messkammer beabstandet angeordnet wird, so dass der Zufluss der Probenflüssigkeit zum Cantileverpaar keinen oder nur einen geringen strömungstechnischen Einfluss auf die Detektion des Analyten nimmt. Der Abstand entspricht hierbei etwa der Höhe der Messkammerwand.

Wenn das Cantileverpaar in der Messkammerwand angeordnet wird, dann kann die Vorrichtung besonders kompakt gestaltet werden.

Die Messkammer kann als oder am Messkammerdeckel ein Probendepot aufweisen, insbesondere ein nanoporöses Reservoir aufweisen, das dazu eingerichtet ist, die Probe aufzunehmen.

Das Aufnehmen der Probe kann beispielsweise dafür sorgen, dass die Probenflüssigkeit gespeichert wird. Dadurch kann insbesondere die Probenflüssigkeit nach einer gewissen Zeit das den Cantilevern zugängliche Messkammervolumen verlassen, so dass das Messkammervolumen mit einer neuen Probenflüssigkeit gefüllt werden kann. Bevorzugt kann das Probendepot als kapillare Pumpe fungieren und somit die konzentrationsabhängige Diffusionsvariation des Teilchenstroms minimieren.

Das nanoporöse Reservoir weist hierbei Kavitäten in der Größenordnung von Nanometern auf. In diesen Kavitäten kann sich demnach die Probenflüssigkeit absetzen, so dass die Probenflüssigkeit in dem Reservoir gespeichert wird. Indem das Reservoir nanoporös ist, kann es dementsprechend viele solcher Kavitäten aufweisen. Dadurch kann insbesondere ein Großteil des Volumens des nanoporösen Reservoirs zur Aufnahme von Probenflüssigkeit verwendet werden. Durch die nanoporösen Kavitäten kann das Probendepot bevorzugt als kapillare Pumpe fungieren und die Probenflüssigkeit in die Messkammer pumpen.

Die Messkammer kann die Probenflüssigkeit in das Probendepot ableiten.

Dadurch kann ein konstanter Fluss der Probenflüssigkeit erreicht werden. Bei einem solchen konstantem Fluss der Probenflüssigkeit, also ohne Obstruktion und ohne plötzliches Durchströmen der Probenflüssigkeit, kann eine quantitative Auswertung des Vorkommens des Analyten oder der Analyte besonders einfach realisiert werden.

Die Vorrichtung kann einen mikroelektronischen Schaltkreis aufweisen, der dazu eingerichtet das elektrische Signal des Cantileverpaars zu empfangen.

Der mikroelektronische Schaltkreis, kann beispielsweise die einzelnen Transducerwiderstände vermessen und/oder die Messwerte zu einer Steuerungseinheit weiterleiten, die die Messwerte in Digitalwerte übersetz und für ein Endgerät aufbereitet. Der mikroelektronische Schaltkreis stellt demnach eine Schnittstelle zwischen der analogen Messwertproduktion des Cantileverpaars und dem Endgerät dar, auf dem die Messdaten ausgewertet werden.

Zu diesem Zweck kann der mikroelektronische Schaltkreis insbesondere analog/digital (A/D) Wandler umfassen.

Der mikroelektronische Schaltkreis kann außerhalb der Messkammer, insbesondere um die Messkammer herum, angeordnet sein.

Dies hat den Vorteil, dass das Cantileverpaar beliebig um die Messkammer herum angeordnet werden kann und die elektronischen Leistungen von den Transducern bis zu dem mikroelektronischen Schaltkreis möglichst kurzgehalten werden können. Dadurch wird insbesondere das elektronische Rauschen reduziert.

Der mikroelektronische Schaltkreis kann auch ein Deep Embedded System bereitstellen, das Signale verarbeiten kann.

Der mikroelektronische Schaltkreis kann an eine Antenne angeschlossen sein, wobei die Antenne um die Messkammer herum verlaufen kann.

Die Antenne kann hierbei aus einer oder mehreren Leiterbahnen bestehen, die um die Messkammer herumgeführt werden können. Durch die Größe der Antenne und die Anzahl an beteiligten Leiterbahnen kann die Induktivität der Antenne eingestellt werden.

Beispielsweise kann der mikroelektronische Schaltkreis eine Batterie aufweisen, die durch die Antenne induktiv geladen werden kann.

Die Batterie kann beispielsweise die Strom- und Spannungsversorgung der Transducer bereitstellen, so dass eine Messung des elektrischen Widerstands der Transducer erfolgen kann. Durch das induktive Laden benötigt die Vorrichtung zudem keinen kabelgebundenen Stromanschluss, so dass die Vorrichtung besonders transportabel wird. Anstatt oder zusätzlich zu einer Batterie kann auch ein Akkumulator eingesetzt werden.

Der mikroelektronische Schaltkreis kann über die Antenne ausgelesen werden.

Beispielsweise kann die Antenne als physikalische RFID oder NFC ("Near Field Communication") oder WLAN- oder Bluetooth-Schnittstelle genutzt werden, so dass die Messwerte der Transducer in regelmäßigen zeitlichen Abständen ausgelesen und ausgewertet werden können. Beispielsweise kann eine mit einer NFC-Schnittstelle ausgestatte Vorrichtung per Smartphone ausgelesen werden, wenn die Leseantenne des Smartphones in die Nähe der Antenne der Vorrichtung gebracht wird.

Die Daten können zwischengespeichert werden, wenn keine Kommunikationsverbindung besteht, und dann ausgetauscht werden, wenn die Kommunikationsverbindung wieder hergestellt ist.

Das Cantileverpaar kann aus einem Chip geformt sein.

Dies kann bedeuten, dass der Sensor auf einer Halbleiterstruktur hergestellt wird, die weitergehende Datenverarbeitung der Transducermesswerte beziehungsweise der Ausgabewert der AD-Wandlerlogik zulässt. Insbesondere kann mit Chip auch ein sogenanntes System-On-A-Chip gemeint sein, wobei alle funktionalen Einheiten des Messsystems integral auf einem einzigen elektronischen Bauteil ausgebildet werden.

Die Referenz- und Testcantilever können Si3N4, SiO2, Si3N4/SiO2, SiC, Si umfassen oder aus Si bestehen oder ein Polymer oder ein Metall mit dünner Oxidschicht, beispielsweise Aluminiumoxid umfassen. Gleichermaßen können auch die Basen beziehungsweise die Gesamtbasis die den genannten Materialien umfassen. Die Basen und die Referenz- und Testcantilever können auch einteilig miteinander aus den genannten Materialien durch herkömmliche Herstellungsprozesse, wie sie bei der Bearbeitung von Wafern bekannt sind, hergestellt werden.

Durch die siliziumbasierten Referenz- und Testcantilever lassen sich aus der Halbleiterindustrie bekannte Herstellungsverfahren verwenden, sodass die Herstellung erfindungsgemäßer Sensoren in einem großen industriellen Maßstab ermöglicht wird. Polymere lassen sich ebenfalls in großem industriellem Maßstab herstellen und weisen den Vorteil auf, dass deren Materialeigenschaften in großem Umfang vorbestimmt werden können.

Die Messkammer kann als Kavität des Chips ausgebildet sein.

Dies hat den Vorteil, dass der Chip sowohl das Cantileverpaar als auch das Messkammervolumen bereitstellt. Dementsprechend ist die mechanische Verbindung zwischen Cantileverpaar und Messkammer fix vorgegeben und die Messkammer und das Cantileverpaar können zusammen gefertigt werden, wodurch die Produktionskosten reduziert werden können. Insbesondere kann hierbei besonders einfach realisiert werden, dass das Cantileverpaar auf der Wand der Messkammer angeordnet ist.

Der Chip kann applikationsspezifische integrierte Schaltungen aufweisen.

Applikationsspezifische integrierte Schaltungen ("ASICs") können hierbei eine vordefinierte Funktion übernehmen, die den Eingangswert von den Transducern mit der gegebenen Funktion in einen Ausgabewert umwandeln.

Bevorzugt können so auch weitere Sensoren integriert werden, wie beispielsweise Gyroskope, Temperatursensoren und optische Sensoren zur Bestimmung der Blutgaswerte und anderer Parameter wie der Pulsrate.

Der Chip kann eine Vielzahl von Cantileverpaaren aufweisen, die insbesondere um die Kavität herum angeordnet sind, wobei jedes Cantileverpaar spezifisch für die Detektion eines Analyten ausgebildet ist.

Beispielsweise kann ein erstes Kantieverpaar den Lactatwert vermessen, ein zweites Cantileverpaar kann den Blutsauerstoffwert vermessen, ein drittes Cantileverpaar kann einen Entzündungswert messen, ein viertes Cantileverpaar kann einen weiteren Biomarker vermessen, und so weiter. Dadurch wird erreicht, dass eine Vielzahl von Analyten gleichzeitig in der Probenflüssigkeit bestimmt wird, so dass mit einer einzigen Vorrichtung eine komplexe Analyse des Vitalzustandes des Probenkörpers erfolgen kann.

Der Chip kann auf dem mikroelektronischen Schaltkreis angeordnet sein. Insbesondere können die Transducer über Bond-Drähte mit den applikationsspezifischen integrierten Schaltungen verbunden sein. Insbesondere können die Bauteile des mikroskopischen Schaltkreises in einem ersten Fertigungsschritt gefertigt werden und die Chipherstellung kann getrennt davon erfolgen. Somit kann die elektronische Auswertung und die Messwertaufnahme für sich optimiert werden.

Die Probenzuleitungsfläche der Messkammer kann eine Vielzahl von Kapillaren aufweisen.

Dadurch kann ein verstärkter und bevorzugt kontrollierter Zufluss der Probenflüssigkeit in das Messvolumen erreicht werden. Zudem kann durch eine Vielzahl von Kapillaren die Vorrichtung besser an dem Probenkörper angebracht werden und beispielsweise seitliches verrutschen vermieden werden, so dass Hautirritationen vermieden werden.

Die oben genannte Aufgabe wird außerdem durch eine Sensorvorrichtung zur Detektion eines Analyten in einer Probe mit den Merkmalen des Anspruchs 13 gelöst. Entsprechend wird eine Sensorvorrichtung zur Detektion eines Analyten in einer Probe vorgeschlagen, wobei die oben beschriebene Vorrichtung in einem Emplastrum angeordnet ist, das Emplastrum auf eine Schicht aufgeklebt wird, die Kapillare in die Schicht eindringt und/oder durch die Schicht dringt und dort eine Probe entnimmt und der Messkammer zuführt.

Ein Emplastum ist hierbei eine haftklebrige Schicht, mit der die Vorrichtung auf der Schicht des Probenkörpes befestigt werden kann. Beispielsweise kann die Schicht Haut sein, bevorzugt die Haut eines Landtieres sein, besonders bevorzugt die Haut eines Säugetieres sein, ganz besonders bevorzugt die Haut eines Menschen sein. Es kann aber auch sein, dass der Probenkörper der Körper eines Fisches ist.

Die Vorrichtung kann in einer Tasche des Emplastrums angeordnet werden, so dass die Probenzulauffläche parallel zur haftklebrigen Schicht des Emplastrums verläuft. Die Probenzulauffläche ist liegt hierbei frei, so dass die Kapillaren nicht durch die Tasche des Emplastrums verdeckt werden.

Durch eine solche Ausgestaltung kann die Vorrichtung sicher am Probenkörper gehalten werden, während die Probenflüssigkeit entnommen und analysiert wird.

Beispielsweise kann die Messelektronik mit Batterie und/oder Akku lösbar mit der Messkammer und der Kapillare verbunden sein. Dadurch kann die Messkammer mit der Kapillaren aus hygienischen Gründen ausgetauscht werden, während die Messelektronik wieder- und/oder weiterverwendet werden kann.

Die Diagonale der Sensorvorrichtung kann zwischen 10mm und 15mm betragen und die Höhe kann zwischen 2mm und 3mm betragen.

Durch den kleinen Formfaktor ist es möglich, dass der Benutzer, beispielsweise ein Mensch die Sensorvorrichtung unauffällig tragen kann und durch dabei einen hohen Tragekomfort erfährt. Die Bewegungen des Menschen oder des Tieres werden hierbei nicht oder nur wenig eingeschränkt.

Bevorzugt kann so vermieden werden, dass die Sensorvorrichtung an Kleidung hängen bleibt und es wird nur eine vergleichsweise kleine Hautfläche irritiert, womit beispielsweise ein damit auftretender Juckreiz minimiert werden kann.

Die Vorrichtung und insbesondere die mindestens eine Kapillare können durch eine lösbare, insbesondere eine wasserlösliche Versiegelungsschicht versiegelt sein, die vor dem Aufbringen des Emplastrums erstmals gelöst wird. Dadurch kann insbesondere die Kapillare beim Transport sauber, frei und durchlässig gehalten werden.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine schematische Darstellung der erfindungsgemäßen Vorrichtung;
- Figur 2A, B, C, D, E: eine schematische Darstellung eines Cantileverpaars und dessen Funktionsweise;
- Figur 3: eine weitere schematische Darstellung der erfindungsgemäßen Vorrichtung;
- Figur 4A, B: schematische Darstellungen der erfindungsgemäßen Sensorvorrichtung;
- Figur 5A, B: weitere schematische Darstellungen der erfindungsgemäßen Sensorvorrichtung; und
- Figur 6: eine schematische Darstellung der Verwendung einer Erfindungsgemäßen Vorrichtung in beziehungsweise an einer Lebensmittelverpackung.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

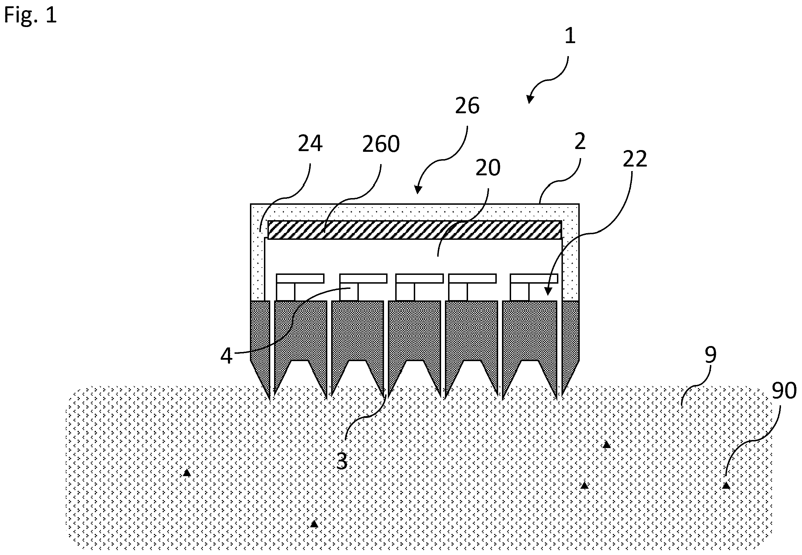

In Figur 1 ist schematisch eine erfindungsgemäße Vorrichtung 1 gezeigt. Die Vorrichtung 1 umfasst eine Messkammer 2, die ein Messkammervolumen 20 aufweist, welches von einer Probenzulauffläche 22, einer Messkammerwand 24 und einem Messkammerdeckel 26 begrenzt wird.

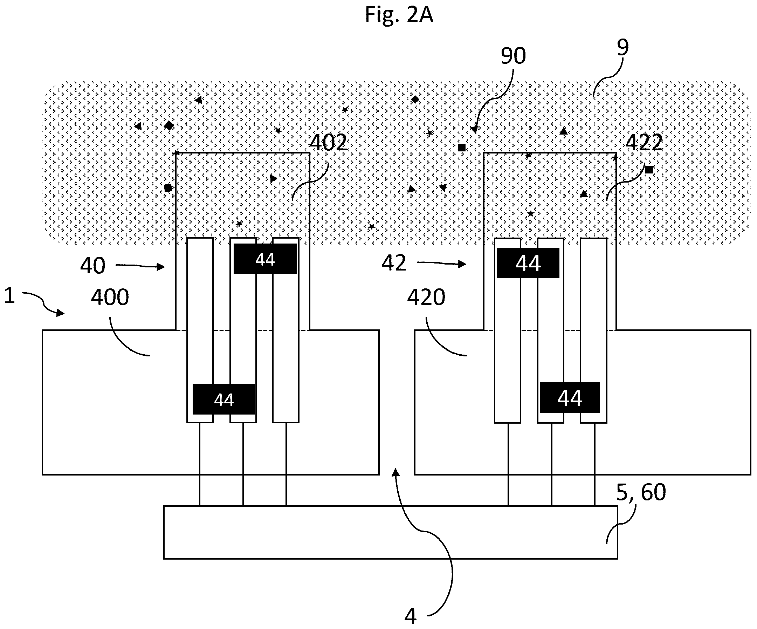

In der Probenzulauffläche 22 sind mehrere Kapillaren 3, hier in Form von Mikronadeln, eingebracht, die eine Probenflüssigkeit 9 aus einem Probenkörper entnehmen und in das Messkammervolumen 20 transportieren können. In dem Messkammervolumen 20 sind verschiedene Cantileverpaare 4 angeordnet, die sowohl einen Referenzcantilever 42 als auch einen Testcantilever 40 umfassen (in Figuren 2A bis 2E beispielsweise gezeigt) und die weiter unter näher beschrieben werden.

Die Kapillaren 3 leiten die Probenflüssigkeit 9 zu den Cantileverpaaren 4, wobei der in der Probenflüssigkeit 9 vorliegende, hier sehr schematisch angedeutete, Analyt 90 in der Probe eine spezifische Verbiegung beziehungsweise Veränderung der Oberflächenspannung der Cantilever 40, 42 (siehe beispielsweise Figuren 2A bis 2E) hervorruft, je nachdem, ob der Analyt 90 mit dem Testcantilever 40 wechselwirken kann oder nicht. Aus dieser Verbiegung oder Veränderung der Oberflächenspannung kann mittels Dehnungsmessstreifen, beziehungsweise mittels Transducern 44, ein elektrisches Messignal erzeugt werden, so dass die relative Verbiegung der Cantilever 40, 42 detektiert werden kann und somit ein Vorkommen eines Analyten 90 detektiert werden kann.

Zusätzlich ist in der Figur 1 ein optionales Probenreservoir 260 gezeigt, welches in dieser Ausführungsform an dem Messkammerdeckel 26 angeordnet ist. Das Probenreservoir 260 ist hierbei in der Lage, die Probenflüssigkeit 9 aus der Messkammer 2 aufzunehmen und zu speichern. Insbesondere kann das Probendepot durch die Nanoporen als Pumpe der Probenflüssigkeit dienen. Dadurch kann die Probenflüssigkeit 9 innerhalb des Messkammervolumens 20 ausgetauscht werden, so dass dem mindestens einen Cantileverpaar 4 ein konstanter Flüssigkeitsstrom mit neuer Probenflüssigkeit 9 zugeführt werden kann. Dementsprechend kann eine zeitabhängige und quantitative sowie qualitative Detektion des Analyten 90 erfolgen.

Für einen kontinuierlichen Zufluss von Probenflüssigkeit 9 aus dem Probenkörper in das Messkammervolumen 20 kann die Kapillare 3 eingesetzt werden, die durch Diffusionskräfte und/oder Kapillarkräfte einen stetigen Nachschub an Probenflüssigkeit 9 in das Messkammervolumen 20 leitet. Um die Stärke und die Größe des Zuflusses einzustellen kann hierbei die Anzahl an Kapillaren 3 in der Probenzulauffläche 22 eingestellt werden. Typischerweise kann durch mehrere Kapillaren 3 auch mehr Probenflüssigkeit 9 in das Messkammervolumen 20 transportiert werden.

In den Figuren 2A bis 2E ist die grundlegende Funktionsweise des Cantileverpaars 4 gezeigt. Das Cantileverpaar 4 umfasst in Figur 2A einen Testcantilever 40, der eine Basis 400 sowie einen verformbaren Teil 402 aufweist und einen Referenzcantilever 42, der eine Basis 420 und einen verformbaren Teil 422 aufweist. Auf den Cantilevern 40, 42 sind Transducer 44 angeordnet, die die Verbiegung der Cantilever 40, 42 durch Wechselwirkung mit dem Analyten 90 ein elektronisches Messsignal umwandeln. Dieses elektronische Messignal kann beispielsweise durch den mikroelektronischen Schaltkreis 5 und/oder die applikationsspezifischen integrierten Schaltungen 60 registriert und weiterverarbeitet werden.

Die Vorrichtung 1 hat die Aufgabe, das Vorkommen und bevorzugt die Menge des Vorkommens eines Analyten 90 in einer Probe 9 anzuzeigen. In jedem Fall soll mit der Vorrichtung 1 die Probe 9 mit Hinblick auf das Vorkommen und/oder eine Konzentration und/oder eine Menge des Analyten 90 untersucht werden. Zu diesem Zweck ist auf den Testcantilever 40 eine Rezeptorschicht aufgebracht, mit der ein Analyt 90 in Wechselwirkung treten kann, beziehungsweise eine Rezeptorschicht die den Analyten 90 adsorbieren oder absorbieren kann.

Sofern die Probe 9 einen Analyten 90 aufweist, kann dieser also mit der Rezeptorschicht in Wechselwirkung treten. Dies kann dazu führen, dass sich die Oberflächenspannung des mit der Rezeptorschicht belegten Abschnitts des verformbaren Teils 402 des Testcantilevers 40 ändert, was zu einer Verformung des verformbaren Teils 402 des Testcantilevers 40 führt.

Jedoch kann es bereits aufgrund der Wechselwirkung mit der Probenflüssigkeit 9 mit den Cantilevern 40, 42 zur Registrierung einer Verformung kommen, beispielsweise indem lediglich die Oberflächenspannung der Probenflüssigkeit 9 auf den verformbaren Teil 402 des Testcantilevers 40 wirkt und diesen verformt. Für eine solche Verformung ist demnach nicht das Vorhandensein eines Analyten 90 verantwortlich.

Um die Größe dieser Grundeinwirkung der Probe 9 auf den Testcantilever 40 festzustellen, wird gleichzeitig mit dem Testcantilever 40 der Referenzcantilever 42 mit der Probe 9 in Kontakt gebracht. Zu diesem Zweck weist der Referenzcantilever 42 eine Referenzschicht auf, mit der ein Analyt 90 nicht in Wechselwirkung treten kann, beziehungsweise eine Referenzschicht, die den Analyten 90 nicht adsorbieren oder absorbieren kann. Hierbei soll eine Wechselwirkung nur mit dem Analyten 90 vermieden werden, um eine Differenzierung zum Messsignal des Testcantilevers 40 zu ermöglichen.

Indem sowohl der Testcantilever 40 als auch der Referenzcantilever 42 mit der Probe 9 in Wechselwirkung treten, wechselwirken beider Cantilever 40, 42 in ähnlicher Art und Weise mit der Probe 9. Hierbei ist jedoch der Unterschied, dass der Testcantilever 40 über seine Referenzschicht zusätzlich mit einem eventuell vorhandenen Analyten 90 in Wechselwirkung treten kann. Dementsprechend unterscheiden sich die Messsignale der Transducer 44, sofern ein Analyt 90 in der Probe 9 vorkommt. Über die Größe des Unterschieds der Messsignale kann demnach im einfachsten Fall auf die Menge des Vorkommens des Analyten 90 in der Probe 9 geschlossen werden.

Um den Unterschied bereits messtechnisch herauszuarbeiten, können die Transducer 44 auf den verformbaren Teilen 402, 422 und den Basen, 400, 420 der Cantilever 40, 42 in einer Vollbrücke verschaltet werden, so dass das elektronische Messignal in einem Vergleich der Verhältnisse der Widerstandswerte der Transducer 44 besteht.

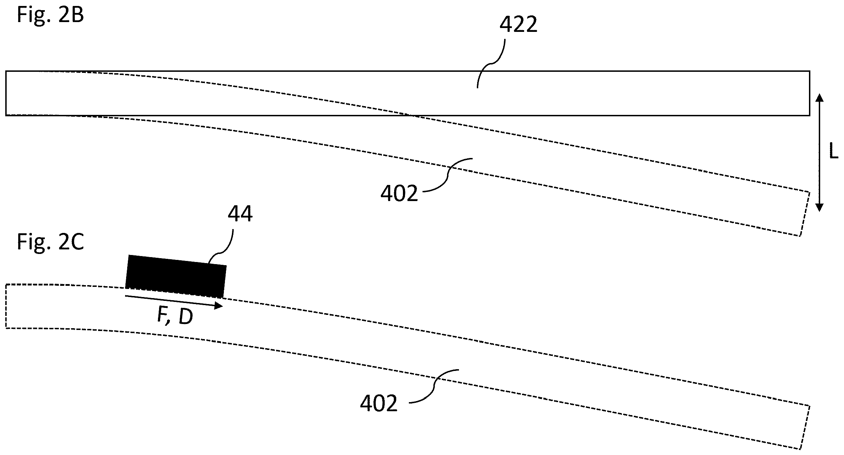

In Figur 2B ist der Vergleich der verformbaren Teile 402, 422 der Referenz-und Testcantilever 42, 40 (siehe Figur 2A) bei einer Verformung und Längsdehnung gezeigt. Der verformbare Teil 422 des Referenzcantilevers 42 weist eine obere Oberfläche und eine untere Oberfläche auf. Ebenso weist der verformbare Teil 402 des Testcantilevers 40 eine obere Oberfläche und eine untere Oberfläche auf. Sofern ein Analyt 90 der Probe 9 mit dem Testcantilever 40, beziehungsweise mit der Rezeptorschicht in Wechselwirkung tritt, findet eine Verformung des verformbaren Teils 402 vom ortsfesten Teil (der in die Basis des Testcantilevers übergeht) hin zum frei beweglichen Teil des verformbaren Teils 402 statt. Die gezeigte Auslenkung L ist hierbei gegeben durch die relative Auslenkung zwischen dem verformbaren Teil 422 des Referenzcantilevers 42 und dem verformbaren Teil 402 des Testcantilevers 40 aufgrund der Wechselwirkung mit dem Analyten 90.

Die Verformung des verformbaren Teils 402 des Testcantilevers 40 ist in Figur 2C gezeigt. Ursächlich hierfür ist, dass sich die obere Oberfläche und die untere Oberfläche des Testcantilevers 40 unterschiedlich stark dehnen. Aufgrund der großen Dehnung D an der oberen Oberfläche, kann ein darauf aufgebrachter Transduktor eine Dehnungskraft F registrieren. Die registrierte Dehnungskraft F kann hierbei durch dem Transducer 44 in ein elektronisches Signal umgewandelt werden beziehungsweise ein vorhandenes elektronisches Signal, beispielsweise eine anliegende Spannung, beeinflussen. Dies kann beispielsweise dadurch geschehen, dass der Transducer 44 den Widerstand ändert, sofern er eine Dehnungskraft F erfährt, die wiederum in einer Dehnung des Transducers 44 resultiert.

Der Transducer 44 könnte auch eine Stauchung der Oberfläche, auf der er angeordnet ist, detektieren. In den gezeigten Ausführungsformen sind die Transduktoren aber immer an Oberflächen angeordnet, bei denen eine Dehnung erwartet wird, also insbesondere an den oberen Oberflächen und/oder den unteren Oberflächen.

Durch die sich auf der Unterseite und der Oberseite des Cantilevers unterscheidenden Oberflächenspannungen kommt es entsprechend zu der beschriebenen Verformung oder Dehnung des Cantilevers.

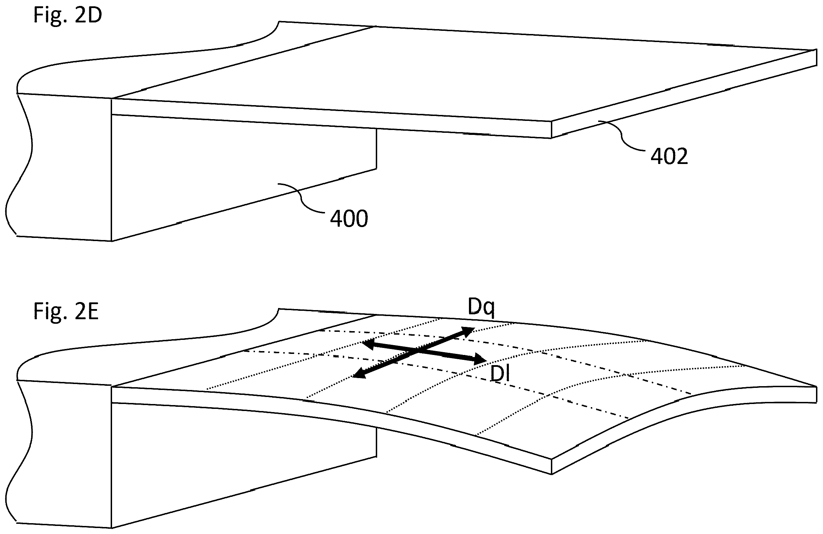

Insbesondere spielt die Ausrichtung der Transducer relativ zur Ausrichtung der Cantilever 40, 42 eine wichtige Rolle. In Figur 2D ist beispielsweise ein unausgelenkter Cantilever 40 gezeigt. Kommt der Cantilever 40 in Kontakt mit dem Analyten 90, so ändert sich die Oberflächenspannung und es kommt zu einer Verformung des verformbaren Teils 402 des Cantilevers 40. Der verformbare Teil 402 erfährt so beispielsweise eine Verformung senkrecht zur Basis 400, wie in Figur 2E gezeigt. Dies geht mit einer Längsausdehnung DI der oberen Oberfläche einher. Gleichzeitig findet eine Verformung parallel zur Basis 400, beziehungswiese zur Biegekante statt, die mit einer Querausdehnung Dq der oberen Oberfläche einhergeht. Durch die Geometrie des Cantilevers 40 kann festgelegt werden, entlang welcher Richtung eine größere Dehnung D bewirkt wird. Insbesondere kann der Transducer entlang dieser Richtung ausgerichtet werden, um ein besonders großes Messsignal zu erzeugen.

Durch eine Überhöhung einer mechanischen Dehnung am Ort des Transducers 44 kann das vom Transducer 44 ermittelte Signal noch weiter verbessert werden. Eine solche Überhöhung kann beispielsweise durch die Anordnung und Form der Elektroden erreicht werden.

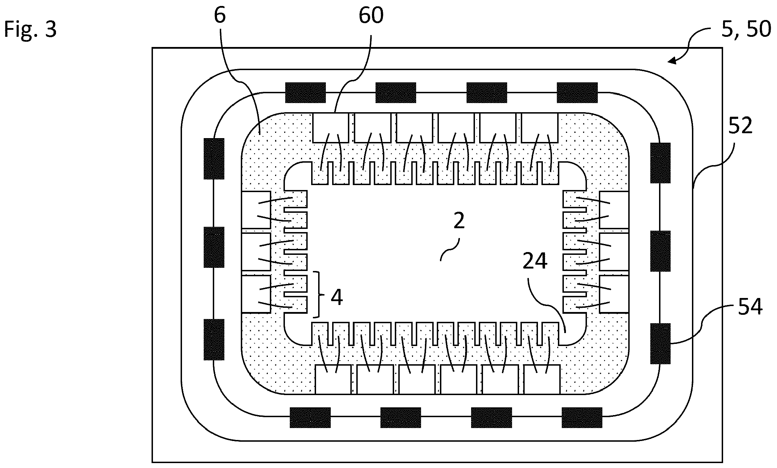

Figur 3 zeigt die Draufsicht auf eine Ausführungsform der Vorrichtung 1. Hierbei sind die Cantileverpaare 4 auf einem gemeinsamen Chip 6 geformt. Zusätzlich sind auf dem Chip applikationsspezifische integrierte Schaltungen 60 aufgebracht. Insbesondere sind in dem Chip 6 eine Vielzahl von Cantileverpaaren 4 ausgeformt und um eine Kavität herum angeordnet. Die Kavität wird am Rand durch die Messkammerwand 24 begrenzt, die beispielsweise durch den Chip 6 gebildet wird. Somit stellt der Chip 6 nicht nur die Cantileverpaare 4 bereit, sondern auch die Messkammerwände 24 mit der das Messkammervolumen begrenzt wird. Zudem sind die Cantileverpaare 4 auf oder an der Messkammerwand 24 angeordnet.

Der Chip 6 ist auf dem mikroelektronischen Schaltkreis 5 angeordnet, welcher beispielsweise aus einem Printable Circuit Board 50 (PCB) gefertigt ist. Printable Circuit Boards können beispielsweise über Photolithographie einfach und in großer Stückzahl hergestellt werden.

Der mikroelektronische Schaltkreis 5 enthält weitere mikroelektronische Elemente 54, wie beispielsweise eine Batterie oder einen Prozessor und einen Speicher zum Speichern von Messwerten. Der mikroelektronische Schaltkreis 5 weist ebenfalls Leiterbahnen auf, die die mikroelektronischen Elemente 54 miteinander verbindet. Der mikroelektronische Schaltkreis 5 ist um den Chip 6 herum und somit insbesondere um das Messkammervolumen 20 (siehe Figur 1) angeordnet. Zusätzlich weist der mikroelektronische Schaltkreis 5 eine Antenne 52 auf, über die beispielsweise die Batterie des mikroelektronischen Schalkreises 5 geladen werden kann, oder aber zur Kommunikation mit dem Prozessor und dem Speicher genutzt werden kann. Beispielswiese können über die Antenne 52 die Analytmesswerte der Cantileverpaare 4 ausgelesen werden.

Es ist aber auch möglich, dass über ein Lesegerät ein Strom in der Antenne 52 induziert wird, die den Messstrom für die Transducer 44 bereitstellt. Dann kann die Vorrichtung 1 ohne Batterien eingesetzt werden. Ein solches Prinzip ist etwa im Bereich der Near Field Communication (NFC) bekannt, bei dem beispielsweise die Daten von (batterielosen) Kreditkarten über einen Schaltkreis ausgegeben werden, der über eine vom Lesegerät induzierte Spannung mit Energie versorgt wird. Dies hat den Vorteil, dass die Vorrichtung 1 keine eigene Energiequelle benötigt und nur dann "aufgeweckt" wird, wenn auch tatsächlich ein Lesegerät zum Auslesen der Analyse bereitgestellt ist.

Alternativ oder ergänzend zu der in der Figur 3 gezeigten Ausgestaltung, in der alle Cantileverpaare 40, 42 auf einem gemeinsamen Chip 6 angeordnet sind, können einzelne Cantileverpaare 40, 42 auch auf separaten Chips 6 angeordnet sein. Entsprechend können so viele Chips wie Cantileverpaare 40, 42 vorgesehen sein, die dann um die zentrale Kavität herum angeordnet sind.

Der Chip 6 beziehungsweise die Chips können auch an einer Aufnahme in der Messkammerwand 24 gehalten sein, wobei der oder die Chips dann die Messkammerwand nicht ausbilden, sondern diese von einem separaten Gehäuse ausgebildet ist.

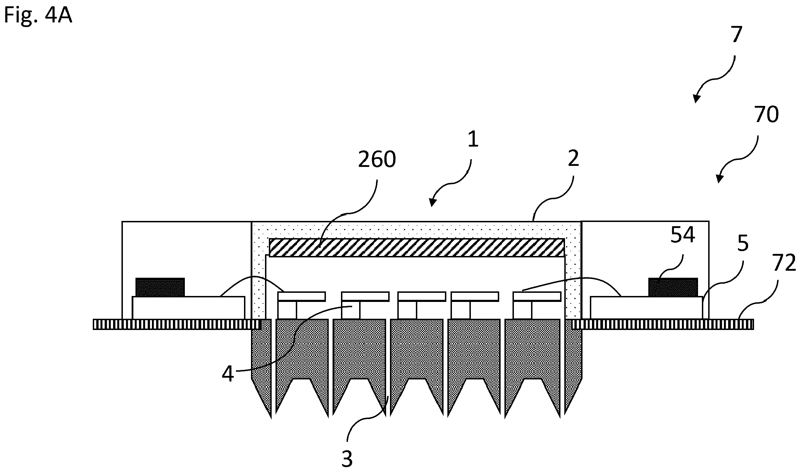

Figur 4A zeigt eine Sensorvorrichtung 7, die eine Vorrichtung 1 umfasst. Ein Emplastrum 70 der Sensorvorrichtung 7 weist eine haftklebrige Schicht 72 auf. Diese haftklebrige Schicht 72 kann auf einen Probenkörper, beispielsweise auf die Haut eines Menschen, aufgeklebt werden. Dabei dringen die Kapillaren 3 in die Haut ein, beispielsweise bis zur Dermis der Haut, und können von dort eine Probenflüssigkeit 9 (siehe Figur 1) in Form einer Körperflüssigkeit, beispielsweise Lymphe oder Intergewebsflüssigkeit oder Schweiß, in die Messkammer 2 leiten. In der Messkammer 2 sind eine Vielzahl von Cantileverpaaren 4 angeordnet, welche die Körperflüssigkeit 9 auf bestimmte Analyten 90 hin untersuchen. Das Emplastrum 70 umschließt hier die gesamte Vorrichtung 1 inklusive der mikroelektronischen Schaltkreise 5 und der mikroelektronischen Bauteile 54. Die Cantileverpaare 4 sind hierbei beispielsweise über Bonddrähte mit dem mikroelektronischen Schaltkries 5 verbunden.

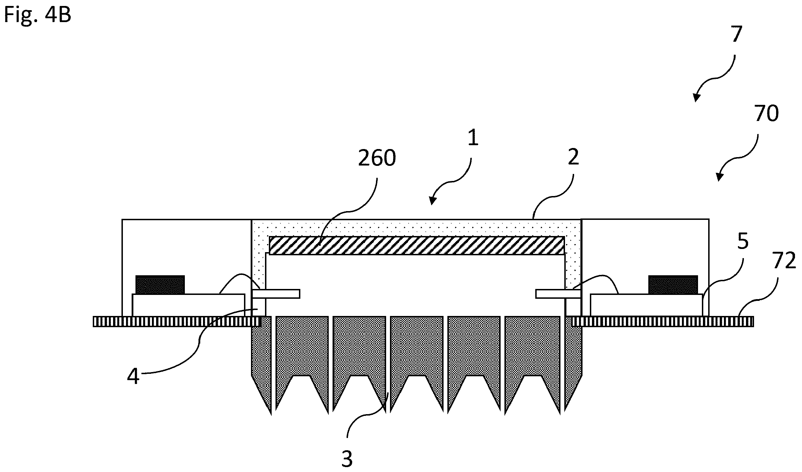

Figur 4B zeigt die Sensorvorrichtung 7, mit dem Unterschied, dass die Cantilever an der Messkammerwand 24 (Figur 3) angeordnet sind.

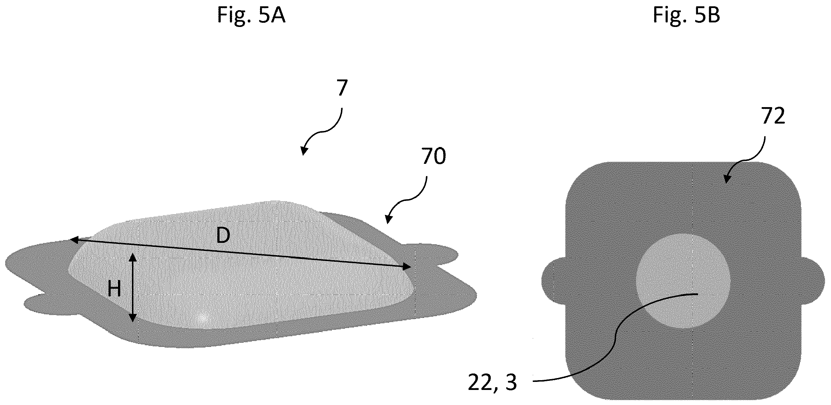

Figur 5A zeigt eine graphische Außenansicht der Sensorvorrichtung 7. Insbesondere umschließt das Emplastrum 70 die Vorrichtung 1. Insbesondere kann das Emplastrum 70 die Vorrichtung wasserdicht verschließen, so dass die Vorrichtung durch von außen auftreffende Flüssigkeiten, beispielsweise Dusch- oder Badewasser oder Regen, keinen Schaden nimmt.

Die Diagonale D der Sensorvorrichtung kann hierbei beispielsweise zwischen 10mm und 15mm betragen und die Höhe H kann beispielsweise zwischen 2mm und 3mm betragen, um ein komfortables Tragen der Sensorvorrichtung zu gewährleisten. Zudem können die Kanten abgerundet sein, so dass das Emplastrum über einen flachen Aufbau verfügt und das Emplastrum nicht an Kleidung hängen bleibt.

In Figur 5B ist entsprechend die Unterseite der Sensorvorrichtung 7 gezeigt. Die Kapillaren 3 der Probenzuleitungsfläche sind von außen zugänglich, so dass durch ein Verkleben des Emplastrums 70 auf der Haut mit der Klebefläche 72, die Kapillaren 3 in die Haut eindringen können, um von dort Probenflüssigkeit 9 zu entnehmen und dem Messkammervolumen 20 zuzuführen.

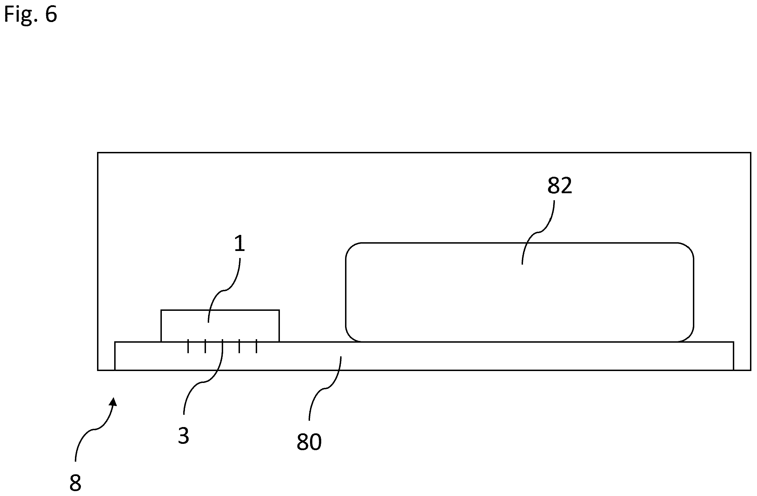

In Figur 6 ist ein weiterer Anwendungsbereich der Vorrichtung 1 gezeigt. Beispielsweise kann die Vorrichtung 1 in der Lebensmittelindustrie eingesetzt werden, beispielsweise in Lebensmittelverpackungen 8. Die Verpackung 8 umschließt das Lebensmittel 80, das beispielsweise auf einer Saugeinlage 82 angeordnet ist, um austretende Flüssigkeiten, beispielsweise Fleischsaft, Gemüsesaft oder Fruchtsaft aufzufangen. Solche Lebensmittel weisen oft eine kurze Haltbarkeit auf, so dass ein schneller Verzehr unabdingbar ist, um eine mögliche Gesundheitsgefährdung zu vermeiden. Mit der Vorrichtung 1 ist es möglich den austretenden Saft über die Saugeinlage 80 aufzufangen und über die Kapillare 3 der Vorrichtung 1 zuzuführen und zu analysieren und beispielsweise auf Analyten, wie Pilze oder Keime hin zu untersuchen.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezuqszeichenliste

- 1: Vorrichtung
- 2: Messkammer
- 20: Messvolumen
- 22: Probenzuleitungsfläche
- 24: Messkammerwand
- 26: Messkammerdeckel
- 260: Probendepot
- 3: Kapillare
- 4: Cantileverpaar
- 40: Testcantilever
- 400: Basis
- 402: verbiegbarer Teil
- 42: Referenzcantilever
- 420: Basis
- 422: verbiegbarer Teil
- 44: Transducer
- 5: mikroelektronischer Schaltkreis
- 50: PCB
- 52: Antenne
- 54: mikroelektronische Bauteile
- 6: Chip
- 60: applikationsspezifische integrierte Schaltung
- 7: Sensorvorrichtung
- 70: Emplastrum
- 72: Klebefläche
- 8: Verpackung
- 80: Saugeinlage
- 82: Lebensmittel
- 9: Probe
- 90: Analyt

## Patentansprüche

1. Vorrichtung (1) zur Detektion mindestens eines Analyten (90) in einer Probe (9), umfassend mindestens eine Kapillare (3), mindestens eine Messkammer (2) und mindestens ein Cantileverpaar (4),
wobei die Kapillare (3) dazu eingerichtet ist, eine Probe (9) zu entnehmen und der Messkammer (2) zuzuführen,
wobei die Messkammer (2) dazu eingerichtet ist, die Probe (9) zu sammeln und dem Cantileverpaar (4) zur Verfügung zu stellen, und
wobei das Cantileverpaar (4) einen Referenzcantilever (42) und einen Testcantilever (40) umfasst,
wobei der Testcantilever (40) zur selektiven Aufnahme des Analyten (90) aus der Probe (9) eingerichtet ist und wobei der Referenzcantilever (42) zur selektiven Nichtaufnahme des Analyten (90) aus der Probe (9) eingerichtet ist,
wobei die selektive Aufnahme des Analyten (90) durch den Testcantilever (40) und die selektive Nichtaufnahme durch den Referenzcantilever (42) eine relative Verformung und/oder eine relative Veränderung der Oberflächenspannung des Testcantilevers (40) zum Referenzcantilever (42) verursacht, und
wobei durch die relative Verformung und/oder die relative Veränderung der Oberflächenspannung des Testcantilevers (40) und des Referenzcantilevers (42) der Analyt detektiert wird.

2. Vorrichtung (1) nach einem der vorherigen Ansprüche, **dadurch gekennzeichnet, dass** die Detektion des Analyten (90) in der Probe (9) kontinuierlich, bevorzugt zeitaufgelöst, durchgeführt wird.

3. Vorrichtung (1) nach einem der vorherigen Ansprüche, **dadurch gekennzeichnet, dass** der Analyt (90) ein Biomarker ist und die Probe (9) eine Körperflüssigkeit ist.

4. Vorrichtung (1) nach einem der vorherigen Ansprüche, **dadurch gekennzeichnet, dass** die Kapillare (3) die Probe (9) der Messkammer (2) über Diffusionskräfte und/oder Kapillarkräfte zuführt und/oder dass die Kapillare (3) einen Durchmesser von weniger als unter 200µm aufweist, bevorzugt von weniger als 100µm aufweist, und eine Länge zwischen 500µm und 5000µm aufweist und/oder dass die Kapillare (3) eine Mikronadel ist.

5. Vorrichtung (1) nach einem der vorherigen Ansprüche, **dadurch gekennzeichnet, dass** die mindestens eine Messkammer (2) ein Messvolumen (20) umschließt, wobei das Messvolumen (20) durch eine Probenzuleitungsfläche (22) mit der mindestens einen Kapillare (3), einer daran anschließenden mindestens einen Messkammerwand (24) und einem Messkammerdeckel (26) definiert ist, wobei bevorzugt die Probenzuleitungsfläche (22) der Messkammer (2) eine Vielzahl von Kapillaren (3) aufweist.

6. Vorrichtung (1) nach einem der vorherigen Ansprüche, **dadurch gekennzeichnet, dass** das Cantileverpaar (4) auf oder in der Messkammerwand (24) angeordnet ist.

7. Vorrichtung (1) nach einem der vorherigen Ansprüche, **dadurch gekennzeichnet, dass** die mindestens eine Messkammer (2) als oder am Messkammerdeckel (26) ein Probendepot (260) aufweist, insbesondere ein nanoporöses Reservoir aufweist, das dazu eingerichtet ist, die Probe (9) aufzunehmen, wobei bevorzugt das nanoporöse Reservoir als Pumpe der Probenflüssigkeit fungiert.

8. Vorrichtung (1) nach einem der vorherigen Ansprüche, **gekennzeichnet durch** einen mikroelektronischen Schaltkreis (5), der dazu eingerichtet ein elektrisches Signal von dem Cantileverpaar (4) zu empfangen.

9. Vorrichtung (1) nach Anspruch 8, **dadurch gekennzeichnet, dass** der mikroelektronische Schaltkreis (5) außerhalb der Messkammer (2), insbesondere um die Messkammer (2) herum, angeordnet ist.

10. Vorrichtung (1) nach einem der Ansprüche 8 oder 9, **dadurch gekennzeichnet, dass** der mikroelektronische Schaltkries (5) an eine Antenne (52) angeschlossen ist, wobei die Antenne (52) bevorzugt um die Messkammer (2) herum verläuft und/oder dass der mikroelektronische Schaltkreis (5) eine Batterie und/oder einen Akkumulator aufweist (54), die bevorzugt durch die Antenne (52) induktiv geladen werden kann und/oder dass der mikroelektronische Schaltkreis (5) über die Antenne (52) ausgelesen werden kann.

11. Vorrichtung (1) nach einem der vorherigen Ansprüche, **dadurch gekennzeichnet, dass** das Cantileverpaar (4) aus oder auf einem Chip (6) geformt ist.

12. Vorrichtung (1) nach Anspruch 11, **dadurch gekennzeichnet, dass** die Messkammer (2) als Kavität des Chips (6) ausgebildet ist und/oder dass der Chip (6) applikationsspezifische integrierte Schaltungen (60) aufweist und/oder dass der Chip (6) eine Vielzahl von Cantileverpaaren (4) aufweist, die insbesondere um die Kavität herum angeordnet sind, wobei jedes Cantileverpaar (4) spezifisch für die Detektion eines Analyten (90) ausgebildet ist und/oder dass der Chip (6) auf dem mikroelektronischen Schaltkreis (5) angeordnet ist.

13. Sensorvorrichtung (7) zur Detektion eines Analyten (90) in einer Probe (9), wobei eine Vorrichtung (1) nach einem der vorherigen Ansprüche in einem Emplastrum (70) angeordnet ist, wobei das Emplastrum (70) dazu eingerichtet ist, auf eine Schicht aufgeklebt zu werden, so dass die Kapillare (3) in die Schicht eindringt und/oder durch die Schicht dringt und dort eine Probe (90) entnimmt und der Messkammer (2) zuführt.

14. Sensorvorrichtung (7) nach Anspruch 13, **dadurch gekennzeichnet, dass** die Schicht Haut ist, bevorzugt die Haut eines Landtieres oder eines Fisches ist, besonders bevorzugt die Haut eines Säugetieres, ganz besonders bevorzugt die Haut eines Menschen ist, und/oder die Schicht eine Verpackung (8) ist, bevorzugt eine Lebensmittelverpackung ist, besonders bevorzugt die Schicht eine Saugeinlage (80) einer Lebensmittelverpackung ist.

15. Sensorvorrichtung (7) nach Anspruch 13 oder 14, **dadurch gekennzeichnet, dass** die Diagonale (D) der Sensorvorrichtung (7) zwischen 10mm und 15mm beträgt und die Höhe (H) zwischen 2mm und 3mm beträgt.
